# EUROPÄISCHE PATENTSCHRIFT

(11) **EP 0 396 866 B1**
(45) Veröffentlichungstag und Bekanntmachung des Hinweises auf die Patenterteilung: **23.10.1996**
(21) Anmeldenummer: 90104211.9
(22) Anmeldetag: 05.03.1990
(51) Int. Cl.: A61B 6/04, A61B 17/22

(54) **Patientenliege**
Patient support
Support de malade

(30) Priorität: 11.05.1989 DE 3915381
(43) Veröffentlichungstag der Anmeldung: 14.11.1990
(73) Patentinhaber: DORNIER MEDIZINTECHNIK GMBH, D-82101 Germering (DE)
(72) Erfinder: Weiler, Herbert, D-8031 Alling (DE); Buchbauer, Peter, Dipl.-Ing., D-8046 Garching (DE); Hofsäss, Siegfried, Dr., D-8034 Germering (DE)
(74) Vertreter: Landsmann, Ralf, Dipl.-Ing.

(56) Entgegenhaltungen:
- EP-A- 0 255 645
- US-A- 3 751 028
- DORNIER LITHOTRIPTER MFL 500, Firmenprospekt

## Beschreibung

Die Erfindung betrifft eine Patientenliege für eine Vorrichtung zur therapeutischen und/oder diagnostischen Behandlung von Patienten gemäß dem Oberbegriff des Patentanspruchs.

Eine derartige Patientenliege ist aus dem Firmenprospekt 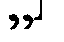 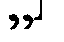 DORNIER LITHOTRIPTER MFL 5000" der Philips Medizin Systeme bekannt. In diesem Prospekt ist eine Patientenliege dargestellt, welche für eine Vorrichtung zur therapeutischen oder diagnostischen Behandlung von Patienten bestimmt ist. Dabei können sowohl die Röntgendiagnostik als auch endo-urologische Therapiemethoden sowie die Stoßwellen-Lithotripsie zum Einsatz kommen. Die Patientenliege besteht aus einem Zentralsegment, das ein Behandlungsfenster aufweist, einem mit diesem fest verbundenen Kopfsegment und einem an der gegenüberliegenden Seite am Zentralsegment ansteckbaren Fußsegment. Die Patientenliege ist mit ihrem Kopfsegment aufeinem Gestell befestigt und kann sowohl in der Vertikalen verstellt als auch in der Horizontalebene verschoben werden.

Der Erfindung liegt daher die Aufgabe zugrunde, eine Patientenliege der eingangs genannten Art bereitzustellen, welche hinsichtlich der Positionierbarkeit des Patienten eine möglichst große Variabilität aufweist.

Diese Aufgabe wird gemäß der Erfindung durch die im kennzeichnenden Teil des Patentanspruchs aufgeführten Merkmale gelöst.

Die erfindungsgemäße Patientenliege besteht demnach aus mehreren aneinandersteckbaren Teilen, wobei ein Zentralsegment vorgesehen ist, das ein abdeckbares Behandlungsfenster hat und ansteckbare Fußsegmente und Kopfsegmente. Das Zentralsegment ist an einem Gerätegestell seitlich auskragend befestigt. Am Zentralsegment können ein Kopfsegment und ein Fußsegment angebracht werden, und zwar sowohl auf der linken als auch auf der rechten Seite Das Fußsegment besteht aus einem Kopfsegment und einem angesteckten Verlängerungssegment. Dadurch wird eine hohe Variabilität erreicht; durch einfaches Umstecken des Verlängerungssegments kann die Liege für die Behandlung der rechten wie der linken Niere umgestellt werden oder von Bauchlage auf Rückenlage verändert werden.

Das Behandlungsfenster kann mit einer Abdeckung verschließbar sein, so daß eine Patientenliege mit durchgehender ebener Fläche gebildet wird. Die Liege kann aus einem Material bestehen, das Röntgenstrahlen im wesentlichen ungeschwächt durchläßt.

Durch die Modularität der erfindungsgemäßen Patientenliege können statt des Fußsegments Unterschenkelauflagen und/oder Miktionsbecken angebracht (angesteckt) werden, wodurch die Liege für urologische oder chirurgische Behandlungen vorbereitet ist.

Die Erfindung wird anhand von zwei Figuren näher beschrieben.

Beide Figuren zeigen einen Lithotripter mit erfindunsgsgemäßer Patientenliege.

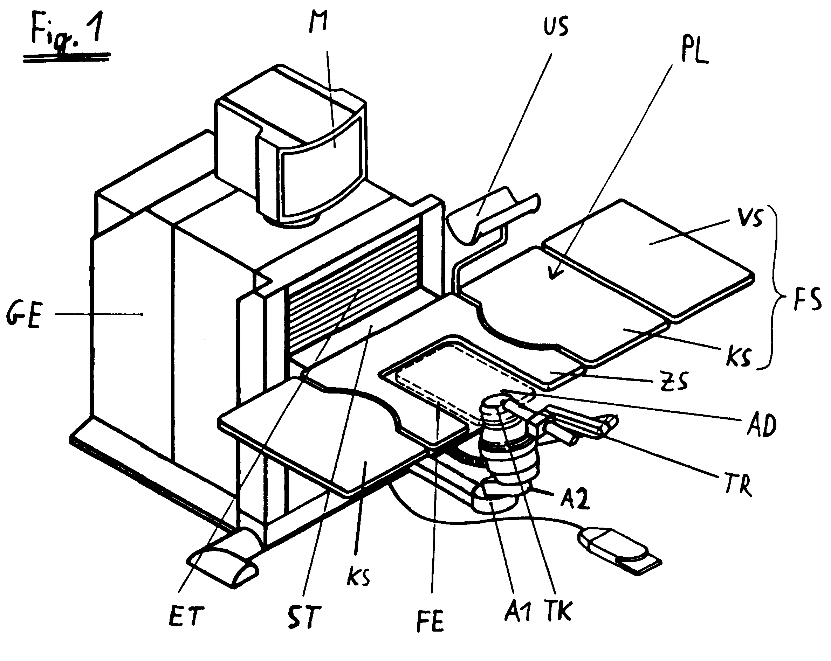

Figur 1 zeigt einen Lithotripter mit dem Gerätegestell GE, der Patientenliege PL, dem Therapiekopf TK und dem Monitor M. Die Patientenliege PL ist hier in ihrer Mitte am Gerätegestell GE über einen Einachsentisch ET nach oben und unten beweglich und über einen Schwimmtisch ST in Horizontalebene verschieblich befestigt. Die Patientenliege PL besteht hier aus einem Zentralsegment ZS mit dem Behandlungsfenster FE, aus dem Kopfsegment KS und aus dem Fußsegment FS, das seinerseits aus einem Kopfsegment KS und einem Verlängerungssegment VS zusammengesteckt ist. Wahlweise können Unterschenkelauflagen angebracht werden (eine Unterschenkelauflage US ist in der Figur 1 gezeigt). Der Therapiekopf TK ist an den Armen A1 und A2 am Geräteträger GE befestigt, befindet sich im Bereich unter dem Behandlungsfenster FE und trägt den Ultraschall-Transducer TR, der zur Ortung dient. Sein Bild ist auf dem Monitor M sichtbar. Die Patientenliege PL ist an ihrem Zentralsegment ZS am Geräteträger GE auskragend befestigt. Das Kopfsegment KS und das Fußsegment FS sind frei wählbar an beiden Seiten des Zentralsegments einsteckbar.

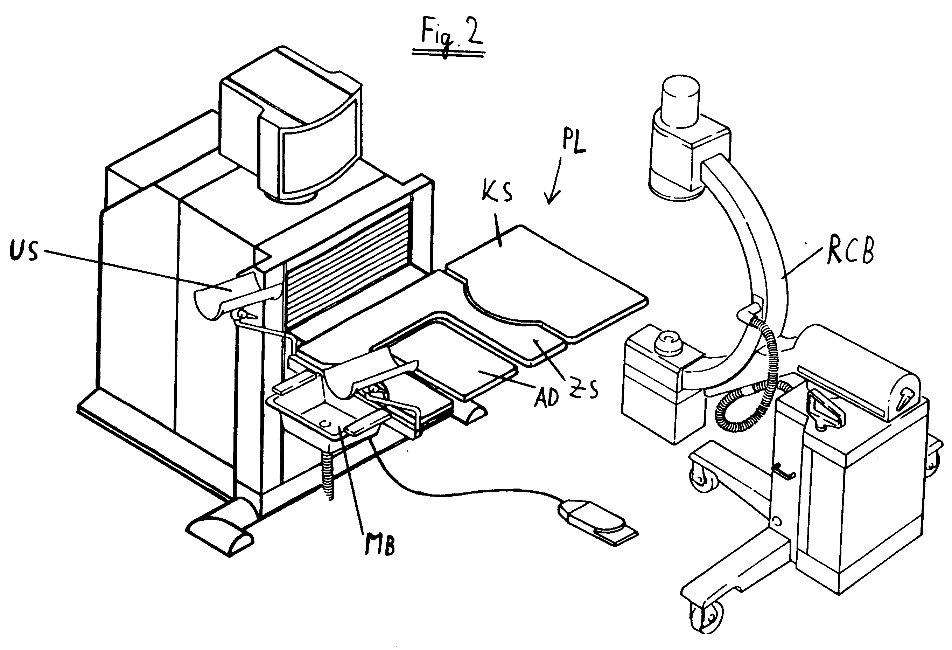

Figur 2 zeigt einen Lithotripter mit einer umgebauten Patientenliege PL. Das Kopfsegment KS befidnet sich jetzt auf der rechten Seite des Zentralsegments ZS. Statt der Fußsegmente sind nun Unterschenkelschalen US und ein Miktionsbecken MB an der linken Seite des Zentralsegments angebracht. Das Behandlungsfenster ist von der Abdeckung AD geschlossen. Der Therapiekopf TK ist in eine Parkposition verschwenkt, so daß der Bereich unter der Patientenliege PL frei zugänglich ist. Daneben ist ein Röntgen-C-Bogen RCB gezeigt, der zur Röntgen-Ortung an die Patientenliege PL gefahren werden kann. Durch die freie Zugänglichkeit kann der Bildverstärker des Röntgen-C-Bogens RCB oder die Röntgenquelle des Röntgen-C-Bogens RCE unter einen beliebigen Bereich des Patientenkörpers geschoben werden. Die Liegenauflagen können in einer Ausführung für Röntgenstrahlen durchlässig sein.

## Patentansprüche

1. Patientenliege (PL) für eine Vorrichtung zur Behandlung von Patienten, welche an einem Gerätegestell (GE) in der Vertikalen verstellbar und in der Horizontalebene verschiebbar befestigt ist, mit einem Zentralsegment (ZS), das ein Behandlungsfenster (FE) aufweist, einem Kopfsegment (KS) und einem ansteckbaren Fußsegment (FS), **dadurch gekennzeichnet,** daß das Zentralsegment (ZS) am Gerätegestell (GE) seitlich auskragend befestigt ist, an zwei einander gegenüberliegenden Seiten des Zentralsegments (ZS) je ein Kopfsegment (KS) und an mindestens einem dieser Kopfsegmente (KS) zur Bildung des Fußsegmentes (FS) ein Verlängerungselement (VS) ansteckbar ist.

## Claims

1. Patient's bed (PL) for a device for the treatment of patients, which is attached to the frame of an apparatus (GE) so as to be vertically adjustable and movable in the horizontal plane, comprising a central segment (ZS) with a treatment window (FE), a head segment (KS) and an attachable foot segment (FS), **characterised in that** the central segment (ZS) is attached to the apparatus frame (GE) so as to be laterally lipped, allowing an attachment of a respective head segment (KS) on two opposite sides of the central segment (ZS) and on at least one of these head segments (KS) an extension element (VS) for forming a foot segment (FS).

## Revendications

1. Table médicale (PL) pour une installation de traitement de malades fixée sur un châssis (GE) avec possibilité de réglage dans la direction verticale et possibilité de coulissement dans le plan horizontal, comportant un segment (ZS) central qui est pourvu d'une fenêtre de traitement (FE), un segment (KS) repose-tête et un segment (FS) repose-pieds, caractérisée par le fait que le segment (ZS) central est fixé latéralement en porte-à-faux au châssis (GE), qu'un segment (KS) repose-tête peut être fixé au segment (ZS) central sur chacun de deux côtés mutuellement en vis-à-vis et qu'un élément prolongateur (VS) peut être fixé à l'un au moins de ces segments (KS) repose-tête pour former le segment (FS) repose-pieds.
